# EUROPEAN PATENT APPLICATION

(11) **EP 3 228 317 A1**
(43) Date of publication of application: **11.10.2017**
(21) Application number: 17165571.5
(22) Date of filing: 07.04.2017
(51) Int. Cl.: A61K 36/062, A01N 43/68, A01N 57/16, A61K 31/17, A61K 31/53, A61P 33/00

(54) **EQUINE PARASITE TREATMENT METHODS AND COMPOSITIONS**

(30) Priority: 07.04.2016 US 201662319718 P
(71) Applicant: Smartpak Equine LLC, Plymouth, MA 02360 (US)
(72) Inventor: Fleming, Casey, Rehoboth, MA Massachusetts 02769 (US); Gray, Lydia, Elburn, IL Illinois 60119 (US); Gisholt, Paal, Duxbury, MA Massachusetts 02332 (US)
(74) Representative: Manitz Finsterwald Patentanwälte PartmbB

(57) **Abstract**

New compositions and methods are provided for controlling a parasitic infection in a horse through administration of an effective amount of *D. flagrans.* In certain aspects, multiple, distinct strains of *D. flagrans* are administered. In additional aspects, the amount and treatment regimen of *D. flagrans* administered to a horse is based on one or more pre-identified characteristics of the horse, is combined with additional therapeutic agents for more comprehensive parasite control, or is accomplished via custom made, pre-measured daily dose packages.

## Description

### FIELD

New compositions and methods are provided for controlling parasitic infection in a horse through administration of an effective amount of *D. flagrans.* In certain aspects, multiple, distinct strains of *D. flagrans* are administered. In additional aspects, the amount and treatment regimen of *D. flagrans* administered to a horse is based on one or more pre-identified characteristics of the horse, is combined with additional therapeutic agents for more comprehensive parasite control, or is accomplished via custom made, pre-measured daily dose packages.

### BACKGROUND

Various types of parasites regularly plague horses. Intestinal parasites can pose substantial health issues in horses such as colic, weight loss and unthriftiness. Certain chemicals such as macrocyclic lactones, pyrimidines, benzimidazoles, and praziquantel have been employed as anthelmintic agents to control equine parasitic infections in horses.

These prior agents however have notable shortcomings. Parasite strains resistant to these therapeutics have evolved. Specifically, none of the three chemical classes retain full efficacy against small strongyles. There is a varying degree of resistance to each, from emerging resistance to widespread resistance. In addition, there are no new, safe, and effective anthelmintic agents likely to be approved in the near future.

It thus would be desirable to have new compositions and methods for control of equine parasitic infections.

### SUMMARY

We now provide new compositions and methods for control of parasitic infections in horses, particularly parasitic infections such as small strongyles (equine cyathostomin) and/or large strongyles infections.

In one aspect, compositions and methods of the invention comprise use and administration of effective amounts of the fungal agent *Duddingtonia flagrans* (*D. flagrans*) to a horse in need thereof.

In a preferred aspect, multiple, distinct strains of *D. flagrans* may be administered to a horse. Therapeutic efficacy may be enhanced by administration of multiple strains.

In a further aspect, the administered amount of *D. flagrans* may be pre-selected based on one or more characteristics of the horse.

In another aspect, *D. flagrans* may be combined with additional therapeutic agents.

In the present methods, the administered *D. flagrans* can be administered to a horse or other subject, wherein the administered *D. flagrans* passes through the digestive tract of the subject and germinates or otherwise resides in the subject's manure. The *D. flagrans* then captures infective parasites within the subject's manure and thereby effectively controls or prophylactically treats parasite infection of the subject.

Additionally, *D. flagrans* may be administered in custom made, pre-measured daily dose packages. The packages may be formed from a variety of materials such as plastic or paper and include multiple sections for segregating distinct compositions being administered to a particular horse or other mammal.

Preferably, the administered amount of *D. flagrans* will be packaged based on one or more previously identified characteristics of a horse or other mammal. For instance, the administered amount of *D. flagrans* may be packaged together with additional feed and/or therapeutic agents based on one or more previously identified characteristics of the horse or other mammal.

Thus, a horse owner may utilize and administer a daily, weekly or other periodic package form of *D. flagrans* that has a treatment amount previously selected for the specific horse.

In particularly preferred aspects, *D. flagrans* may be co-administered with one or more additional therapeutic agents, for example one or more anti-parasite agents such as a macrocyclic lactone, a pyrimidine, a benzimidazole or praziquantel. The *D*. *flagrans* can be administered to the subject prior to, concurrently with, or subsequent to the administration of the one or more additional therapeutic agents.

A variety of treatment regimens may be suitably employed. For instance, *D. flagrans* may be administered to a horse together with food. *D. flagrans* may be administered to a horse under varying schedules, for example, once per day; multiple times per day; 1, 2, 3, 4 or 5 times per week; and for extended periods such as for 5, 10, 20, 30, 40, 50 consecutive weeks or more.

The invention also provides feed supplement formulations that comprise *D*. *flagrans*. The formulations may be included in a feed supplement to horses.

In a separate aspect, the invention provides a novel horse treatment composition or supplement. Certain embodiments provide that the treatment composition comprise at least two distinct strains of *D. flagrans.* The formulation of the invention may be in a solid or liquid form. In one embodiment, the formulation is in a pellet form.

In still another aspect, the invention provides a method of treating, preventing and/or controlling a parasite infection condition in a subject such as a horse. In certain embodiments, the method involves administering to the subject a composition or formulation described herein. In certain embodiments, the composition or formulation of the invention is administered together with food (e.g., a feed). The administration can be an oral administration. In a further embodiment, the product is top-dressed on feed.

Other aspects of the invention include a kit which contains a *D. flagrans* composition or formulation described herein. The kit may come with written instructions for administration of the composition or formulation to a subject, such as, a wide variety of animals including, specifically, a horse.

Other aspects of the invention are discussed *infra.*

### DETAILED DESCRIPTION

In one aspect, the invention features provides novel compositions that can be used as a supplement to horses to control parasite infections.

In certain embodiments, a composition of the invention includes an effective amount of *D. flagrans.*

In particular aspects, a composition of the invention includes an effective amount of multiple, distinct strains of *D. flagrans*, for example 2, 3, 4 or more distinct strains of *D. flagrans.*

Exemplary suitable strains of *D. flagrans* include the Brazilian isolate of *D. flagrans* CG 768 as disclosed in A Buzatti et al., Experimental Parasitology, 159 (2015) 1-4; *D. flagrans* strain Troll A, CBS 101616 available from Christian Hansen A/S and as described in The EFSA Journal (2006) 334, 1-8; *D. flagrans* 03/99 from Argentina as described in Federica et al. Parasitol Res (2013) 112:1047-1051; and *D. flagrans* strain CI3 as described in Fernandez et al., Equine Veterinary Journal (1999) 31(6) 488-491.

The invention also provides a method of treating, preventing and/or controlling a parasite infection in a subject. The subject described herein is a mammal, such as a horse. Treatment, prevention and/or control methods of the invention include administering *D. flagrans* to a horse or other subject. The administered *D. flagrans* can pass through the digestive tract of the subject and reside in the subject's manure and therein capture infective parasites. This will reduce the overall parasite population within the subject's environment and thereby prophylactically treat, prevent or otherwise control parasitic infections to which the subject may be susceptible.

### Definitions

Before a further description of the present invention, and in order that the invention may be more readily understood, certain terms are first defined and collected here for convenience.

The term "administration" or "administering" includes routes of introducing a composition, formulation, or product to a subject to perform its intended function. Typically, *D. flagrans* will be administered orally. Other administration routes will be possible, but less typically used. In certain administration protocols, where a further treatment agent is administered together with or otherwise in conjunction with *D. flagrans*, the further treatment agent may be suitably administered by a variety of routes, including orally as well as an injectable (e.g. subcutaneously, intra-muscularly, intravenously) or other method. The preparations (e.g., composition, formulations, or products) are, of course, given by forms suitable for the particular administration route. For example, the preparations may be administered in tablets or capsule form, by a powder, pellets, granules or other form fed separately, or by a powder, pellets, granules or other form top dressed on food or feed.

The composition, formulations, or products can be administered alone, or in conjunction with other nutrients or therapeutic agents, or with a carrier acceptable for use in food/feed industries, or both. The composition, formulations, or products can be administered prior to, simultaneously with, or after the administration of the other nutrients or therapeutic agents.

In this disclosure, "comprises," "comprising," "containing" and "having" and the like can have the meaning ascribed to them in U.S. Patent law and can mean " includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments. "Detect" refers to identifying the presence, absence or amount of the object to be detected.

The term "effective amount" or similar term refers to the amount of an agent required to control parasite infection in an untreated subject. The effective amount of active ingredients used to practice the present invention may vary depending upon the manner of administration, the age, body weight, and general health of the subject. Such amount is referred to as an "effective" amount.

An effective amount of a component or composition delineated herein (*i.e*., an effective dosage) may vary widely and suitably may range from about 1 x 10⁴ to 1 x 10⁷ spores of *D. flagrans* per kilogram of bodyweight of the horse being treated, more typically from about 1 x 10⁵ to 1 or 2 x 10⁶ spores of *D. flagrans* per kilogram of bodyweight of the horse being treated.

In compositions that contain multiple, distinct strains of *D. flagrans*, each strain suitably will be present in an amount of at least 2, 3, 4, 5, 10, 20, 30, 40, or 50 weight percent based on total weight of *D. flagrans* being administered in a dose.

If desired, the dosage is administered one time per day, or multiple times per day such as twice a day. A single daily dose often will be suitable. Alternatively, the dosage is administered one time every two days, one time every three days, one time every four days, one time every five days, one time every six days, or one time per week. It will also be appreciated that the effective dosage of a composition delineated herein used for its intended purpose may increase or decrease over the course of a particular treatment.

### Compositions, formulations and other products

A *D. flagrans* composition used herein can be in a liquid or solid form such as, a powdered, pelleted, or granulated form, or where *D. flagrans* spores may be coated or otherwise applied on other material such as feed.

Further, the invention provides a supplement, which includes a *D. flagrans* composition described herein. Such a supplement may be, for example, used as a feed supplement to a horse which comprises *D. flagrans* as disclosed herein. In certain embodiments, the supplement of the invention may further contain one or more additional nutrients, such as, vitamins, minerals, amino acids, proteins, herbs, fats, or combinations thereof

The invention also provides feed supplement formulations. The formulations may be included in a feed supplement to horses.

The composition or formulation of the invention may include one or more additional biologically active and/or inactive components. Such components can be for example surfactants, disintegrants, diluents, stabilizers, preservatives, buffers, fillers, plasticizers, lubricants, excipients, and etc. Additional components that can be included in a supplement are known to those of ordinary skill in the art.

Although the present description of the invention is generally directed to use of the invention with horses, it should be understood that the invention can be used in the same manner with other animals, including pets and livestock.

### Methods

In one aspect, the invention provides a method of treating, preventing or controlling a parasitic infection in a horse. In certain embodiments, the method involves administering a composition of the invention to the horse simultaneously with or without food (e.g., a feed).

The subject is suitably a horse, but as discussed also may be other animals such as animals of livestock, including, for example, camelids (llamas and alpacas), cattle, sheep, goats, pigs, and the like, as well as pets such as dogs and cats.

The condition that can be prevented and controlled is a symptom or condition associated with a parasitic infection, including small strongyles (equine cyathostomin) and/or large strongyles infections.

In certain embodiments, the subject could have been identified as having one or more of the diseases or disorders described herein, including a parasitic infection such as small strongyles (equine cyathostomin) and/or large strongyles infections. Identification of the diseases or disorders as described herein by a skilled veterinarian or other medical practitioner is routine in the art.

As discussed, according to another embodiment of the invention, the composition of the invention may be combined with other therapeutic agents. These therapeutic agents may be those known effective in alleviating, treating or preventing in the subject a same or different parasitic infections. It is so contemplated that such combinations may, in some cases, provide better results or synergistic effects.

It is intended that the additional therapeutic agent(s) may be administered to the subject prior to, concurrently with, or subsequent to the administration of the composition of the invention.

The therapeutic agents can be, for example, small molecule therapeutics, natural products, and/or extracts that have been known and/or used in the industry for the treatment of parasitic infections. For example, the therapeutic agent may be a macrocyclic lactone, a pyrimidine, a benzimidazole or praziquantel. Further the therapeutic agent may be diatomaceous earth. In another aspect, the therapeutic agent could be an insect growth regulator (IGR) such as cyromazine or diflubenzuron.

Additionally, the therapeutic agent could be parasitic insects such as parasitic wasps used to control the fly population on a farm.

It is so contemplated that the composition of the invention and the other therapeutic agent(s) may be administered to the subject through the same route or by different routes.

In accordance with the invention, the composition can be administered to the subject once or multiple times a day. In certain embodiments, the composition is administered once, twice, three or four times a day. Single-dose daily administration often will be preferred.

### Kits and dosages

The invention also provides a kit, which contains a composition or formulation as described above. The kit may come with written instructions for administration of the composition or formulation to a subject including, such as, a wide variety of animals. In certain aspects, the subject is an animal including a mammal (such as livestock and pets), especially, a horse.

The written instructions may also include instructions for mixing the composition to be mixed with an animal feed. Furthermore, the written instructions may generally include information for use of the composition in preventing and controlling a parasitic infection in a subject, including small strongyles (equine cyathostomin) and/or large strongyles infections.

In other embodiments, the instructions include at least one of the following: description of the one or more components of the composition; dosage schedule and administration and prevention and control of a condition to be controlled including a parasitic infection; precautions; warnings; indications; counter-indications; adverse reactions; animal pharmacology; clinical studies; and/or references. The instructions may be directly printed on a container or packaging (when present), or as a label applied to a container or other packaging, or as a separate sheet, pamphlet, card or folder supplied in or with a container or other packaging.

In particular embodiments, the kit instructions may include DFAH (Dosage Form Animal Health) product label components, such as one or more of the following: *Product Facts*: active ingredients *Duddingtonia flagrans*; inactive ingredients, as may be present in the composition; *Cautions*: safe use in pregnant animals or animals intended for breeding has not been proven, if animal's condition worsens or does not improve, stop product administration and consult your veterinarian; *Directions for Use*: give one scoop daily or other amount; *Warnings*: for animal use only, keep out of the reach of children and animals, in case of accidental overdose, contact a health professional immediately, this product should not be given to animals intended for human consumption.

The following non-limiting examples are illustrative of the invention.

### Example 1 -- Composition

A preferred composition contains Troll A isolate of *D. flagrans* spores admixed with feed supplement.

### Example 2 - Administration

The composition of Example 1 is administered via a daily dose of the *D. flagrans*/feed supplement mixture daily at a dose of in an amount of 5 x 10⁵ spores per kg bodyweight of horse. Such daily doses continue without interruption year-round. Dosing may be curtailed during non-grazing season months if desired.

Further advantageous embodiments:
1. A method for treating or controlling a parasitic infection in a mammal, comprising:
   administering to the mammal an effective amount of *D. flagrans* selected for the mammal to control a parasitic infection.
2. A method for treating or controlling a parasitic infection in a mammal, comprising:
   administering to the mammal an effective amount of multiple, distinct strains of *D. flagrans.*
3. The method of item 1 or 2 wherein the mammal is a horse.
4. The method of item 1 or 2 wherein the mammal is a livestock animal.
5. The method of any one of items 1 through 4 wherein the administered *D. flagrans* passes through the digestive tract of the mammal, resides in the subject's manure and therein captures infective parasites.
6. The method of any one of items 1 through 5 wherein the mammal has been identified as susceptible to or suffering from a small strongyles (equine cyathostomin) and/or large strongyles infection.
7. The method of any one of items 1 through 6 wherein the administered amount of *D. flagrans* has been selected based on one or more characteristics of the mammal.
8. The method of any one of items 1 through 7 wherein the administered *D. flagrans* had been packaged based on one or more previously identified characteristics of the mammal.
9. The method of any one of items 1 through 8 wherein the administered *D. flagrans* had been packaged together with additional feed and/or therapeutic agents based on one or more previously identified characteristics of the mammal.
10. The method of any one of items 1 through 9 wherein the one or more additional therapeutic agents are administered to the mammal in conjunction with the *D. flagrans.*
11. The method of any one of items 1 through 10 wherein the *D*. *flagrans* and/or wherein the one or more additional therapeutic agents are administered to the mammal through custom made, pre-measured daily dose packages.
12. The method of any one of items 9 through 11 wherein the one or more additional therapeutic agents are selected from a macrocyclic lactone, a pyrimidine, a benzimidazole, pyraziquantel, diatomaceous earth, an insect growth regulator, and/or parasite wasps.
13. The method of any one of items 1 through 12 wherein the *D. flagrans* is administered to the mammal together with food.
14. The method of any one of items 1 through 14 wherein the *D. flagrans* is administered to the mammal once per day.
15. The method of any one of items 1 through 13 wherein the *D. flagrans* is administered to the horse multiple times per day.
16. The method of any one of items 1 through 15 wherein the *D. flagrans* is administered to the horse multiple times per week for at least 30 consecutive weeks.
17. A composition for administration to a horse or other mammal comprising multiple, distinct strains of *D. flagrans.*
18. A packaged composition for administration to a horse or other mammal comprising an effective amount of one or more distinct strains of *D. flagrans.*
19. The composition of item 17 or 18 wherein the *D. flagrans* is packaged in an amount based on one or more previously identified characteristics of the mammal.
20. The composition of any one of items 17 through 19 wherein the *D. flagrans* and additional feed and/or therapeutic agents are segregated within a unitary packaging.

### INCORPORATION BY REFERENCE

The entire contents of all patents, published patent applications and other references cited herein are hereby expressly incorporated herein in their entireties by reference.

Those skilled in the art will recognize, or be able to ascertain using no more than routine experimentation, numerous equivalents to the specific procedures described herein. Such equivalents are considered to be within the scope of this invention and are covered by the following claims.

## Claims

1. Use of *D. flagrans* for treatment or control of a parasitic infection in a mammal, wherein the *D. flagrans* is selected for the mammal.

2. Use of multiple, distinct strains of *D. flagrans* for treatment or control of a parasitic infection in a mammal.

3. Manufacture of a medicament for treatment or control of a parasitic infection in a mammal, comprising *D. flagrans,* wherein the *D. flagrans* is selected for the mammal.

4. Manufacture of a medicament for treatment or control of a parasitic infection in a mammal, comprising multiple, distinct strains of *D*. *flagrans*.

5. The use or medicament of any one or claims 1 through 4 wherein the mammal is a livestock animal.

6. The use or medicament of any one of claims 1 through 4 wherein the mammal is a horse.

7. The use or medicament of any one of claims 1 through 6 wherein the *D. flagrans* is packaged based on one or more previously identified characteristics of the mammal.

8. The use or medicament of any one of claims 1 through 7 wherein the *D. flagrans* and/or wherein one or more additional therapeutic agents are in custom made, pre-measured daily dose packages.

9. The use or medicament of any one of claims 1 through 8 wherein an insect growth regulator in addition to D. flagrans are used or in the mediacament.

10. The use or medicament of claim 9 wherein the insect growth regulator is cyroamazine and/or diflubenzuron.

11. Use of *D. flagrans* in combination with an insect growth regulator for treatment or control of a parasitic infection in a mammal.

12. The use of claim 11 wherein the insect growth regulator is cyroamazine and/or diflubenzuron.

13. The use of claim 11 or 12 wherein the mammal is a horse.

14. A composition for administration to a horse or other mammal comprising: 1) *D. flagrans* and 2) an insect growth regulator.

15. The composition of claim 14 wherein the insect growth regulator is cyroamazine and/or diflubenzuron.
